(19) Europäisches Patentamt
European Patent Office
Office européen des brevets

(11) **EP 2 241 240 B1**

(12) **EUROPÄISCHE PATENTSCHRIFT**

(45) Veröffentlichungstag und Bekanntmachung des Hinweises auf die Patenterteilung:
**16.11.2011 Patentblatt 2011/46**

(51) Int Cl.:
*A47L 15/00* (2006.01)    *A47L 15/46* (2006.01)
*A61G 9/02* (2006.01)    *B08B 3/00* (2006.01)

(21) Anmeldenummer: **09005409.9**

(22) Anmeldetag: **16.04.2009**

(54) **Reinigungsverfahren mit verbesserter Langzeit-Hygienewirkung**

Cleaning method with improved long-term hygienic effect

Procédé de nettoyage doté d'un effet hygiénique de longue durée amélioré

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(43) Veröffentlichungstag der Anmeldung:
**20.10.2010 Patentblatt 2010/42**

(73) Patentinhaber: **MEIKO Maschinenbau GmbH & Co. KG**
**77652 Offenburg (DE)**

(72) Erfinder:
• **Braun, Markus**
**77656 Offenburg (DE)**

• **Peukert, Thomas, Dr.**
**77815 Bühl (DE)**

(74) Vertreter: **Isenbruck, Günter et al**
**Isenbruck Bösl Hörschler LLP**
**Eastsite One**
**Seckenheimer Landstrasse 4**
**68163 Mannheim (DE)**

(56) Entgegenhaltungen:
**WO-A-2006/053634    US-B1- 6 615 850**

## EP 2 241 240 B1

**Beschreibung**

Gebiet der Erfindung

Die Erfindung betrifft ein Verfahren und eine Vorrichtung zur Reinigung eines Reinigungsguts sowie ein entsprechendes Computerprogramm. Das Reinigungsverfahren bewirkt insbesondere eine Sicherstellung einer thermischen Hygienewirkung, insbesondere eine verbesserte Langzeit-Hygienewirkung. Insbesondere dient das Verfahren zur Kompensation des sogenannten "lag of regrowth-Effekts". Derartige Reinigungsverfahren und Reinigungsvorrichtungen können beispielsweise in den Naturwissenschaften, der Technik, der Medizin oder der Pflegetechnik eingesetzt werden, um unterschiedliche Arten von Reinigungsgut einer Reinigung und Hygienisierung, bis hin zur Desinfektion, zu unterziehen. Anwendungsbeispiele sind die Geschirrspültechnik oder auch die Reinigung von Pflegegeschirr.

Stand der Technik

Aus dem Stand der Technik sind in vielen Bereichen des täglichen Lebens, der Technik, der Naturwissenschaften und der Medizin und/oder dem Pflegebereich Reinigungsvorrichtungen für eine Vielzahl von zu reinigenden Objekten bekannt. Als Beispiele, welche das mögliche Anwendungsspektrum der vorliegenden Erfindung jedoch nicht beschränken, können Geschirrspülmaschinen genannt werden, welche im Haushaltsbereich oder beispielsweise im gewerblichen Bereich als Einkammer- oder Mehrkammergeschirrspülmaschinen in Benutzung sind. Als weiteres Beispiel sind Reinigungsvorrichtungen für medizinische Geräte und/oder Pflegegeräte zu nennen, insbesondere Behältnisse, bei welchen hohe Flüssigkeitsmengen als Abfall anfallen. Beispielsweise sind hier Reinigungsdesinfektionsgeräte zu nennen, welche beispielsweise für die Reinigung von Steckbecken, Bettpfannen, Urinflaschen, Nachtgeschirr oder ähnlichen Behältnissen und Instrumenten aus dem medizinischen Bereich oder Pflegebereich eingesetzt werden können.

Viele Reinigungsvorrichtungen dienen der Reinigung von Reinigungsgut, welches unmittelbar oder mittelbar mit möglicherweise keimbelasteten Materialien in Verbindung kommt. Als Beispiel sind hier Objekte zu nennen, welche mittelbar oder unmittelbar mit dem menschlichen Körper in Kontakt geraten und welche somit als Überträger von Krankheiten, insbesondere Infektionen, dienen können und bei denen somit ein besonderer Wert auf eine ausreichende Hygienisierung, d. h. eine ausreichende Keimreduktion, bis hin zu einer Desinfektion zu legen ist.

Die Messung und Sicherstellung eines Hygienegrades und/oder einer Keimreduzierung sind Gegenstand zahlreicher bekannter Verfahren und Normen. So ist insbesondere bei einer Einwirkung von feuchter Wärme auf das Reinigungsgut ein Zusammenhang von Temperatur und Zeit auf die Keimreduzierung in Mehrtankgeschirrspülmaschinen untersucht worden. So legt beispielsweise die DIN 10510 für Deutschland Mindestanforderungen hinsichtlich Temperatur, Reinigerkonzentration und Dauer zwischen dem ersten Kontakt des zu reinigenden Gutes mit der Spülflüssigkeit bis zum Verlassen der Maschine fest. Grundlage dieser Norm ist die Keimreduktion von definiert angeschmutzten Prüfkörpern nach dem Reinigungsprozess über sogenannte Abklatschuntersuchungen. Als Testkeim bzw. Organismus wird in diesem Test E. faecium ATCC 6057 verwendet.

Die Prüfung der Hygienesicherheit von Mehrtankspülmaschinen beim Endabnehmer wird in der Regel über Abklatschuntersuchungen und die Bestimmung der Keimzahl im Spülwasser des letzten Spültanks vorgenommen. Nachteilig an diesem Verfahren ist jedoch der Umstand, dass die Prüfung der Keimreduktion vor Ort beim Kunden nach dieser Norm nur mit großem Aufwand durchgeführt werden kann. Ein weiterer Nachteil dieser Norm ist der Umstand, dass die gleiche Keimreduktion beispielsweise auch bei einer kürzeren Kontaktzeit, jedoch bei höheren Temperaturen in den einzelnen Behandlungszonen erreicht werden könnte. Dies lässt die genannte Norm jedoch nicht zu.

Dementsprechend existieren Normen, welche die Hygienisierungswirkung der Reinigungsgeräte mittels kumulierter Wärmeäquivalente bestimmen. Unter Wärmeäquivalenten werden dabei allgemein, wie unten noch näher ausgeführt wird, Integrale oder Summen über einen zeitlichen Verlauf der Temperatur oder eines Funktionswertes einer monoton wachsenden Funktion der Temperatur bezeichnet, mit welcher das Reinigungsgut beaufschlagt wird. Die Temperatur wird dabei üblicherweise nach einer festgelegten Wichtungsfunktion gewichtet bzw. unmittelbar in gewichtete Werte umgewandelt.

So wird in den USA beispielsweise der Zusammenhang von Temperatur und Zeit auf die Keimreduzierung durch das sogenannte NSF3-Standardverfahren beschrieben. Basis dieser Vorgabe ist die aus Versuchen ermittelte Keimreduktion von Tuberkulosebakterien durch die Einwirkung einer Temperatur über die Zeit. Das Einwirken der Temperatur über die Zeit wird dabei als Wärmeäquivalent bezeichnet. Wie viele Wärmeäquivalente bei welcher Temperatur erzielt werden, ist in einer Tabelle in diesem Verfahren niedergeschrieben. Als Mindesttemperatur, aber welcher Wärmeäquivalente gezählt werden, wird dabei nach diesem Standard eine Temperatur von 62 °C (entsprechend 143 °F) angeführt. Für Spülmaschinen gilt dabei üblicherweise, dass zur Erfüllung der geforderten Keimreduzierung mindestens 3600 Wärmeäquivalente nach dieser Vorschrift erreicht werden müssen. Vorteilhaft an diesem Verfahren ist, dass das Verfahren mit relativ wenig Aufwand vor Ort durchgeführt werden kann, um die einwandfreie Funktion einer Geschirrspülmaschine hinsichtlich der thermischen Hygienisierung zu überprüfen.

Auch in Deutschland und Europa existieren entsprechende Normen. Beispielsweise wird für Reinigungsdesinfektionsgeräte in EN ISO 15883-1 ein Verfahren beschrieben, welches zur Beurteilung der Hygienewirkung ebenfalls im Zusammenhang zwischen der Keimreduzierung und der Temperatur über die Zeit heranzieht. Dieser Zusammenhang wird auch als $A_0$-Wert bezeichnet und ist ebenfalls in tabellarischer Form niedergeschrieben bzw. errechnet sich aus einer mathematischen Formel. Der $A_0$-Wert wird dabei im Anhang A dieser Norm näher beschrieben und ist definiert als Zeitäquivalent in Sekunden bei 80 °C, bei dem eine gegebene Desinfektionswirkung ausgeübt wird, und entspricht sinngemäß der Vorgehensweise im NSF3-Standard, jedoch unter Zugrundelegung eines anderen Testkeims.

Im Rahmen der europäischen Normungsarbeit für Desinfektionsverfahren wurde das $A_0$-Konzept entwickelt. Es entstand aus dem Anliegen, die Wirksamkeitskontrolle thermischer Desinfektionsverfahren transparenter und die Freigabe desinfizierter Güter von mikrobiologischen Kontrollkulturen unabhängig zu machen (parametrische Freigabe). Das $A_0$-Konzept geht von der Annahme aus, dass zur reproduzierbaren Inaktivierung einer bestimmten Mikrobenlast eine durch Temperatur und Einwirkzeit definierte Energiemenge nötig ist. Dies ist in Anlehnung an das F-Konzept bei der thermischen Sterilisation medizinischer Güter, bei der allerdings wesentlich höhere Energiemengen nötig sind als bei der Desinfektion. Das $A_0$-Konzept beinhaltet Annahmen, die aufgrund von Erfahrungswerten zur thermischen Abtötung von Mikroben getroffen worden waren. Die experimentellen Grundlagen sind jedoch dürftig und teilweise widersprüchlich. Eine systematische experimentelle Überprüfung der Annahmen des $A_0$-Konzeptes mit verschiedenen Mikrobenklassen wurde bislang noch nicht durchgeführt.

Nach der Norm EN ISO 15883-1 (Reinigungs- und Desinfektionsgeräte - Teil 1: Allgemeine Anforderungen, Definitionen und Prüfungen) wird die thermische Desinfektion mit feuchter Hitze in Reinigungs- und Desinfektionsgeräten (RDG) parametrisch über den $A_0$-Wert definiert und kontrolliert. Dieser repräsentiert die aufgewendete Energie als Produkt aus aufgewendeter Temperatur und Einwirkungszeit. Damit werden biologische Indikatoren als zeitaufwendige und variable "Messlatte" für die Freigabe des Desinfektionsgutes auf weite Strecken entbehrlich und durch Registrierung physikalischer Parameter ersetzt. Ausgangspunkt ist der A-Wert, der ein Zeitäquivalent in Sekunden bei 80°C darstellt, bei dem eine gegebene Desinfektionswirkung erzielt wird. Für das $A_0$-Konzept sind folgende Messeinheiten von Bedeutung:

- Der als dezimale Reduktionszeit oder D-Wert bezeichnete mikrobiologische Parameter als Maß, welches das thermische Absterbeverhalten von Mikroorganismen charakterisiert. Der D-Wert gibt an, welche Zeit zur Abtötung von 90% der Mikroorganismen einer Population bei einer gegebenen Temperatur T notwendig ist, also um die Population auf 10% der Anfangsmenge zu senken. Der D-Wert stellt somit ein Maß für die Hitzesensibilität einer bestimmten Mikroorganismen-Art dar.

- Mittels des so genannten z-Werts wird die Abhängigkeit des D-Werts von der Temperatur T erfasst. Der z-Wert gibt an, welche Temperaturerhöhung notwendig ist, um den D-Wert um 9/10 zu senken, also den gleichen Abtötungseffekt in einem Zehntel der Zeit zu erreichen.

- Der $A_Q$-Wert ist der A-Wert bei Mikroorganismen, deren z-Wert 10°C ist; für das $A_0$-Konzept lautet die Annahme z=100C. Diese Annahme, gemeinsam mit der Grundthese, dass die gleiche Desinfektionswirkung sowohl durch höhere Temperatur und niedrigere Einwirkdauer als auch durch niedrigere Temperatur und entsprechend längere Einwirkdauer erreicht werden kann, ergibt ein gut reproduzierbares und praktikables Konzept für die Handhabung thermischer Desinfektionsverfahren.

In der EN ISO 15883-1 werden für die Praxis je nach Wichtigkeit der Keimfreiheit, $A_0$-Werte von 60, 600 und 3000 vorgeschlagen, wobei ein $A_0$ von 3000 gegenüber einem $A_0$ von 60 die 50-fache Energiemenge bedeutet und eine deutlich bessere Desinfektionswirkung erreicht. In Tabelle 1 sind Beispiele für Temperaturen und Zeiten für die jeweiligen $A_0$-Werte dargestellt.

*Tabelle 1: Zeitäquivalente für verschiedene $A_0$-Werte.*

| Temperatur | $A_0$60 | $A_0$600 | $A_0$3000 |
|---|---|---|---|
| 70°C | 10 min | 100 min | 500 min |
| 75°C | 3,2 min | 32 min | 160 min |
| 80°C | 1 min | 10 min | 50 min |
| 85°C | 0,3 min | 3,2 min | 16 min |
| 90°C | 6 s | 1 min | 5 min |
| 93°C | 3 s | 30 s | 2,5 min |

Aus dem Stand der Technik sind verschiedene Reinigungsvorrichtungen bekannt, welche die thermische Hygienisierung von Reinigungsgut nach einem oder mehreren der Standards überwachen. So beschreibt beispielsweise WO 2006/097294 A1 ein Verfahren zur Beurteilung und Sicherstellung der thermischen Hygienewirkung in einer Durchlaufspülmaschine. Dabei sind in der Durchlaufspülmaschine ein oder mehrere Sensoren dauerhaft angebracht, welche die Temperatur innerhalb einzelner Behandlungszonen erfassen und an eine Maschinensteuerung übermitteln. Die Maschinensteuerung ermittelt die thermische Hygienewirkung über die auf das zu reinigende Gut jeweils einwirkende Temperatur und Zeit anhand von Wärmeäquivalenten. Der Spülprozess kann derart gesteuert werden, dass eine voreingestellte Menge an Wärmeäquivalentwerten auf das zu reinigende Gut übertragen wird.

In DE 10 2007 021 245 A1 wird eine Desinfektionssteuerung durch Zielerregerauswahl vorgeschlagen. Dabei wird eine Information über einen Zielkeim empfangen, mit dem das zu reinigende Objekt kontaminiert sein könnte, und mindestens ein Prozessparameter wird derart gewählt, dass der Zielkeim bei dem Reinigungsprozess mit hoher Wahrscheinlichkeit abgetötet wird. Der Reinigungsprozess wird dann mit dem Prozessparameter durchgeführt.

In der Praxis hat es sich jedoch bei Verwendung von Hygienisierungsverfahren, welche auf der Erfassung von Wärmeäquivalenten basieren, beispielsweise nach einem oder mehreren der genannten Standards, gezeigt, dass die vorhergesagten Hygienisierungen nicht in allen Fällen eintreten. Insbesondere hat es sich gezeigt, dass Reinigungsgut, wie beispielsweise Geschirr, welches nach den theoretischen Vorhersagen der Normen eigentlich einen bestimmten Hygienegrad aufweisen sollte, tatsächlich einen geringeren Hygienegrad aufweist. Zudem hat es sich gezeigt, dass der gemessene Hygienegrad unter Umständen stark von der Zeitdauer abhängen kann, welche zwischen der Hygienisierung und der Messung verstrichen ist.

Aufgabe der Erfindung

Es ist daher eine Aufgabe der vorliegenden Erfindung, ein Reinigungsverfahren und eine Reinigungsvorrichtung bereitzustellen, welche die Nachteile bekannter Vorrichtungen und Verfahren vermeiden. Insbesondere soll eine auch längerfristig anhaltende verbesserte Hygienisierung gewährleistet werden können.

Offenbarung der Erfindung

Diese Aufgabe wird mit der Erfindung mit den Merkmalen der unabhängigen Patentansprüche gelöst. Vorteilhafte Weiterbildungen der Erfindung, welche einzeln oder in Kombination realisierbar sind, sind in den abhängigen Ansprüchen dargestellt.

Es werden ein Verfahren zur Reinigung und/oder Desinfektion von Reinigungsgut sowie eine Reinigungsvorrichtung zur Reinigung und/oder Desinfektion von Reinigungsgut vorgeschlagen, wobei das Verfahren unter Verwendung einer erfindungsgemäßen Reinigungsvorrichtung durchgeführt werden kann und wobei die Reinigungsvorrichtung eingerichtet sein kann, um ein Verfahren gemäß einer der beschriebenen Verfahrensvarianten durchzuführen. Dementsprechend kann beispielsweise für mögliche Ausgestaltungen der Reinigungsvorrichtung auf die Beschreibung möglicher Ausgestaltungen des Verfahrens verwiesen werden und umgekehrt. Das Verfahren und die Vorrichtung können beispielsweise dem Zweck der Reinigung dienen oder beispielsweise dem Zweck der Desinfektion (also der Keimreduktion) oder beiden Zwecken gemeinsam und können dementsprechend eingerichtet sein. Weiterhin wird ein Computerprogramm mit Programmcode vorgeschlagen, welches eingerichtet ist, um das Verfahren in einer oder mehreren der beschriebenen Ausführungsformen durchzuführen, wenn das Programm in einem Computer ausgeführt wird, beispielsweise einem Computer einer Reinigungsvorrichtung, beispielsweise einer Steuerung einer Reinigungsvorrichtung. Unter einem Durchführen kann dabei sinngemäß auch ein Steuern verstanden werden, so dass das Verfahren beispielsweise eingerichtet sein kann, um eine Reinigungsvorrichtung zur Durchführung der einzelnen Verfahrensschritte anzuregen, wenn das Programm in einem Computer der Reinigungsvorrichtung, beispielsweise einem Computer in einer Steuerung der Reinigungsvorrichtung, ausgeführt wird. Das Computerprogramm kann insbesondere auf einem maschinenlesbaren Träger gespeichert sein.

Das vorgeschlagene Reinigungsverfahren basiert auf grundlegenden Überlegungen und Untersuchungen herkömmlicher Standards, welche auf der Erfassung und Kumulierung von Wärmeäquivalenten basieren. So bewertet beispielsweise die konventionelle $A_0$-Wert-Methode die Desinfektionswirkung auf der Basis eines Temperatur-Zeit-Integrals oder von Thermoäquivalenten bzw. kumulierten Thermoäquivalenten. Zählbare, d. h. wirksame Einheiten von Thermoäquivalenten werden dabei ab einer Temperatur von 65 °C erfasst. Nach oben ergibt sich eine Begrenzung in der Regel nur durch praktische und rein technische Restriktionen. Niedrige Temperaturen werden dabei weniger wirksam als höhere bewertet, indem diese einen geringeren Funktionswert einer Wichtungsfunktion erhalten. Die Berechnung der Desinfektionswirkung kann beispielsweise durch Lösen entsprechender $A_0$-Wert-Gleichungen oder durch Integration mit bekannter Temperaturfunktion erfolgen. In der Praxis werden dabei häufig Temperaturäquivalente diskreter Zeitscheiben berechnet und aufsummiert. Dabei wird jedoch allgemein von der Annahme ausgegangen, dass alle Thermoäquivalente, die bei Temperaturen von mindestens 65 °C erfasst werden, grundsätzlich als wirksam zu betrachten sind, wenn auch mit unterschiedlicher Wichtung. Dementsprechend ist es unerheblich, ob ein bestimmtes Thermoäquivalent und ein bestimmter $A_0$-Wert bei höheren oder niedrigeren Temperaturen erreicht wird. Allein die dazu benötigte Zeit, also ein Prozessparameter, bildet die Unterscheidung. Ähnliche Überlegungen ergeben sich auch für kumulierte Thermoäquivalente nach dem NSF3-Standard.

In der Praxis hat sich jedoch bei verschiedenen Untersuchungen gezeigt, dass die Annahme einer Wirksamkeit ab einer für den entsprechenden Thermoäquivalent-Standard vorgegebenen Mindesttemperatur (beispielsweise 65 °C für EN ISO 15883 oder 62 °C (entsprechend 143 °F) für NSF3) für verschiedene Mikroorganismen und Prozessparameter nicht ohne Ausnahme zuzutreffen scheint. So ist in bestimmten Fällen der bereits oben angedeutete Effekt zu verzeichnen, welcher auch als "lag of regrowth" bezeichnet wird. Damit wird ein Phänomen bezeichnet, bei welchem Mikroorganismen, beispielsweise andere Mikroorganismen als diejenigen, die in den genannten Standards verwendet werden, bei bestimmten Temperaturen zunächst in der für die Prozessparameter erwarteten Art und Weise abzusterben scheinen und/oder reduziert zu werden scheinen.

Insofern treffen die Vorhersagen der entsprechenden Standards zunächst zu. Nach Verstreichen einer durchaus auch längeren Ruhezeit, beispielsweise einer Ruhezeit von einigen Stunden bis hin zu einigen Tagen oder sogar einigen Wochen, weisen dieselben Mikroorganismen dann jedoch eine wachsende Vitalität auf und können sich wieder verstärkt vermehren. Insofern zeigt es sich, dass in vielen Fällen die Mikroorganismen zumindest teilweise nicht wirklich absterben, sondern lediglich inaktiviert werden und aufgrund mangelnder Vermehrung ein Absterben suggerieren.

Für die Desinfektionspraxis, beispielsweise in Reinigungsvorrichtungen, bedeutet dies jedoch, dass Reinigungsgut auch bei demselben, nach den Normen bestimmten Reinigungsgrad im Langzeitverhalten erhebliche Unterschiede aufweisen kann. Diese Unterschiede sind dadurch bedingt, dass zwar letztendlich das Reinigungsgut mit derselben kumulierten Anzahl von Thermoäquivalenten beaufschlagt wurde, jedoch möglicherweise bei unterschiedlichen Prozessparametern, d. h. insbesondere unterschiedlichen Temperaturen und mit unterschiedlichen Behandlungszeiten, um zu diesen kumulierten Thermoäquivalenten zu gelangen. Diese Unterschiede bestimmen jedoch ein unterschiedliches Verhalten des Reinigungsguts hinsichtlich des "lag of regrowth-Effekts". So kann insbesondere das Langzeitverhalten von Reinigungsgut, bei welchem ein bestimmter $A_0$-Wert oder ein entsprechender Wert nach dem NSF3-Standard erreicht wurde, sehr wohl abhängig von der während des Desinfektionsprozesses zumindest zeitweise herrschenden Temperatur sein. Weiterhin können auch die konkret zu reduzierenden Mikroorganismen sich in ihrem Langzeitverhalten stark unterscheiden, auch wenn die konkrete Art von Mikroorganismus bei der Berechnung des $A_0$-Wertes berücksichtigt wurde, so dass nach dem Reinigungsprozess zunächst derselbe Hygienisierungsgrad bzw. Desinfektionsgrad vorliegt. Somit kann auch ein genau definierter Hygienisierungsgrad, beispielsweise ein $A_0$-Wert, auch unterschiedliche Hygieneergebnisse repräsentieren, insbesondere hinsichtlich des Langzeitverhaltens.

Aufgrund dieser erfindungsgemäßen Überlegungen basiert die Erfindung darauf, dass Reinigungsprozesse, welche eine Hygienisierungs- und/oder Desinfektionswirkung aufweisen, derart gesteuert werden sollten, dass Gefährdungen durch Verfälschung des Hygieneergebnisses ausgeschlossen werden können. Dabei sollten auch konkrete Erkenntnisse über die Auswirkung von Temperatureinflüssen auf bestimmte Mikroorganismen, insbesondere den oben genannten "lag of regrowth-Effekt", berücksichtigt werden können. Dementsprechend sollte das Verfahren derart durchführbar sein, dass dieses eine Hygienisierung zunächst auf der Basis kumulierter Wärmeäquivalente vornimmt, so dass beispielsweise mindestens ein vorgegebener Desinfektionsgrad, beispielsweise ein vorgegebener $A_0$-Wert erreicht werden kann. Insofern sollte das vorgeschlagene Verfahren herkömmlichen Standards genügen. Gleichzeitig sollte das Verfahren jedoch in der Lage sein, die oben beschriebenen Effekte, welche durch den konkreten zeitlichen Verlauf der Kumulierung von Wärmeäquivalenten und/oder durch die speziellen Eigenschaften bestimmter Mikroorganismen hervorgerufen werden, zu kompensieren. Insofern sollte beispielsweise eine adaptierte Steuerung des Prozesses möglich sein.

Es wird daher ein Verfahren zur Reinigung und/oder Desinfektion eines Reinigungsguts vorgeschlagen, bei welchem das Reinigungsgut mit mindestens einem Reinigungsfluid beaufschlagt wird. Weiterhin wird eine Reinigungsvorrichtung zur Reinigung von Reinigungsgut vorgeschlagen, wobei die Reinigungsvorrichtung mindestens eine Steuerung zur Steuerung eines Reinigungsvorgangs umfasst, wobei die Reinigungsvorrichtung eingerichtet ist, um ein erfindungsgemäßes Verfahren in einer oder mehreren der im Folgenden beschriebenen Formen durchzuführen. Die Steuerung umfasst ein oder mehrere elektronische und/oder elektromechanische Bauteile, darunter eine oder mehrere Datenverarbeitungsvorrichtungen und/oder Netzwerke von Datenverarbeitungsvorrichtungen, beispielsweise einen oder mehrere Computer und/oder Mikro-controller. Weiterhin umfasst die Steuerung Ein- und Ausgabemittel umfassen, um einem Benutzer und/oder einem anderen Computer oder Computernetzwerk die Eingabe von Befehlen und/oder Daten in die Steuerung zu ermöglichen und/oder um Informationen und/oder Daten an den Benutzer und/oder den anderen Computer und/oder das Computernetzwerk ausgeben zu können. Weiterhin kann die Steuerung einen oder mehrere flüchtige und/oder nicht flüchtige Datenspeicher umfassen.

Grundsätzlich können unterschiedlichste Arten von Reinigungsgut eingesetzt werden. Besonders bevorzugt kann es sich bei dem Reinigungsgut um Reinigungsgut handeln, welches direkt oder indirekt mit Speisen und/oder Getränken in Berührung kommt und/oder für die Zubereitung oder Bereitstellung von Speisen und/oder Getränken eingesetzt wird, wie beispielsweise Teller, Tassen, Tabletts, Besteck, Schüsseln, Gläser oder Ähnliches. Dementsprechend kann die Reinigungsvorrichtung beispielsweise als Geschirrspülmaschine ausgestaltet sein und/oder eine derartige Geschirrspülmaschine umfassen. Dabei können Haushaltsgeschirrspülmaschinen oder auch gewerbliche Geschirrspülmaschinen erfindungsgemäß modifiziert werden. Die Geschirrspülmaschine kann beispielsweise als Einkammer- oder Mehrkammergeschirrspülmaschine ausgestaltet sein. Insbesondere können auch Durchlaufgeschirrspülmaschinen, bei welcher das Reinigungsgut mittels einer Transportvorrichtung durch eine oder mehrere Reinigungszonen transportiert wird, erfindungsgemäß ausgestaltet werden.

Alternativ oder zusätzlich können auch andere Arten von Reinigungsgut gereinigt werden. Insbesondere kann das Verfahren somit auch zur Reinigung medizinischer Geräte und/oder Pflegegeräte eingesetzt werden. Insbesondere kann das Pflegegerät Gefäße umfassen, welche für die Aufnahme größerer Mengen (beispielsweise von mehr als 100 ml) menschlicher Ausscheidungen eingerichtet sind, beispielsweise Bettpfannen, Steckbecken, Urinflaschen oder ähnliche Gefäße. Auch eine andere Art von Reinigungsgut ist grundsätzlich reinigbar. Insofern kann die Reinigungsvorrichtung beispielsweise ein oder mehrere Reinigungs- und Desinfektionsgeräte umfassen.

Unter einer Reinigung, welche im Rahmen des genannten Verfahrens und/oder unter Verwendung der genannten Reinigungsvorrichtung in dem Reinigungsvorgang durchgeführt werden kann, kann dabei insbesondere eine Befreiung von anhaftenden Verunreinigungen verstanden werden. Weiterhin beinhaltet das genannte Verfahren, wie unten näher ausgeführt wird, eine Hygienisierungswirkung, wobei unter einer Hygienisierung allgemein eine Keimreduktion verstanden wird, bis hin zu einer Desinfektion, also einer definierten, zumindest nahezu vollständigen Keimabtötung.

Zur Beaufschlagung des Reinigungsguts mit dem mindestens einen Reinigungsfluid wird das Verfahren unter Verwendung einer oder mehrerer Reinigungskammern durchgeführt bzw. die Reinigungsvorrichtung kann eine oder mehrere derartiger Reinigungskammern umfassen, welche ganz oder teilweise als geschlossene Reinigungskammern ausgestaltet sein können, welche jedoch auch ganz oder teilweise als geöffnete Reinigungskammern, beispielsweise Reinigungstunnel, ausgestaltet sein können. Grundsätzlich sind jedoch auch Reinigungsvorrichtungen ohne derartige Reinigungskammern möglich. Zur Beaufschlagung des Reinigungsfluids kommen eine oder mehrere Düsen zum Einsatz. Dabei werden einstrahlige Düsen, mehrstrahlige Düsen oder auch auf andere Weise gestaltete Düsen eingesetzt, welche in der mindestens einen Reinigungskammer aufgenommen sind.

Unter einem Reinigungsfluid kann dabei grundsätzlich ein beliebiges, den Reinigungsvorgang zumindest unterstützendes fluides Medium, d. h. eine Flüssigkeit und/oder ein Gas, verstanden werden. Insbesondere kann das mindestens eine Reinigungsfluid eine wässrige Reinigerlösung umfassen, also eine Lösung, welcher eine Menge mindestens eines Reinigers beigefügt ist. Diesbezüglich kann auf kommerziell erhältliche Reinigerlösungen, wie sie beispielsweise in Reinigungsdesinfektionsgeräten und/oder in Geschirrspülmaschinen eingesetzt werden, verwiesen werden. Alternativ oder zusätzlich kann auch eine Reinigung, beispielsweise mit klarem Wasser erfolgen, beispielsweise in einem Nachspülvorgang in einer Geschirrspülmaschine und/oder in einem Reinigungsdesinfektionsgerät. Dem Frischwasser kann dabei auch beispielsweise ein Klarspüler beigefügt sein, beispielsweise um eine Trocknung von Geschirr, insbesondere Gläsern, zu erleichtern. Eine Klarspülung kann beispielsweise als Pumpenklarspülung und/oder als Frischwasserklarspülung ausgestaltet sein. Wiederum alternativ oder zusätzlich kann das Reinigungsfluid auch einen Dampf umfassen, beispielsweise Wasserdampf. Zu diesem Zweck kann die Reinigungsvorrichtung beispielsweise einen oder mehrere Dampferzeuger umfassen, so dass das Reinigungsgut mit dem Wasserdampf beaufschlagt werden kann. Weiterhin kann der Reinigungsvorgang, welcher auch das erfindungsgemäße Verfahren umfassen kann, auch derart ausgestaltet sein, dass nacheinander mehrere Schritte durchgeführt werden, welche beispielsweise auch mehrere Schritte umfassen können, in welchen das Reinigungsgut mit unterschiedlichen Arten von Reinigungsfluid beaufschlagt wird. So kann beispielsweise in Reinigungsdesinfektionsgeräten nach einer Entleerung der Gefäße zunächst ein Ausspritzen der Gefäße erfolgen, gefolgt von einer optionalen Reinigung mit einer Reinigerlösung, wiederum optional gefolgt von einem Dampfsterilisationsschritt, bei welchem die Gefäße mit Wasserdampf hygienisiert werden, bis hin zu einer Desinfektion bzw. Sterilisation. Bei Geschirrspülmaschinen können beispielsweise optional ein Vorspülen, optional ein Hauptspülen und/oder optional ein oder mehrere Nachspül- oder Klarspülschritte miteinander kombiniert werden.

Allgemein sollte das Verfahren derart durchgeführt werden, dass das Reinigungsgut mittels des mindestens einen Reinigungsfluids mit einer feuchten Wärme beaufschlagt wird, wobei das Reinigungsfluid vorzugsweise eine oberhalb der Raumtemperatur liegende Temperatur aufweist. Beispielsweise kann das Reinigungsfluid eine Temperatur in einem Bereich zwischen 60 °C und 100 °C aufweisen.

Das Verfahren wird derart durchgeführt, dass Wärmeäquivalente erfasst und aufsummiert werden, mit welchen das Reinigungsgut beaufschlagt wird. Diese Erfassung kann beispielsweise während und/oder unmittelbar nach der Beaufschlagung des Reinigungsguts mit dem mindestens einen Reinigungsfluid erfolgen, so dass die Wärmeäquivalente diejenigen Wärmeäquivalente sein können, welche von dem Reinigungsfluid auf das Reinigungsgut übertragen werden.

Unter Wärmeäquivalenten sind dabei allgemein im Rahmen der vorliegenden Erfindung Temperaturen zu verstehen, welchen das Reinigungsgut zu einem Zeitpunkt und/oder über eine Zeitdauer hinweg ausgesetzt ist und welche zur Beurteilung ihrer hygienisierenden Wirkung mit einer Wichtungsfunktion gewichtet werden. Bei dieser Wichtungsfunktion kann es sich insbesondere um eine monoton wachsende Wichtungsfunktion handeln, welche die Temperatur des Reinigungsguts in einen entsprechenden Funktionswert umsetzt, der die keimabtötende Wirkung charakterisiert. Somit kann mittels der Wichtungsfunktion die Temperatur beispielsweise mit monoton wachsenden Wichtungsfaktoren multipliziert werden und/oder die Temperatur kann vollständig durch monoton mit der Temperatur wachsende Funktionswerte ersetzt werden. Als Beispiele derartiger Wichtungsfunktionen kann auf die genannten Standards verwiesen werden. Beispielsweise kann auf den Anhang A des NSF3-Standards verwiesen werden, in welchem Wärmeäquivalente als so genannte HUE-Werte tabellarisch als Funktion der Temperatur angegeben sind und/oder den Anhang A des Teils 1 der Norm EN ISO 15883, in welchem ebenfalls entsprechende Wichtungsfunktionen zur Umrechnung der Temperatur des Reinigungsguts in entsprechende Wärmeäquivalente angegeben sind. Beispiele werden unten näher erläutert.

Die Wärmeäquivalente werden im Rahmen des erfindungsgemäßen Verfahrens aufsummiert, so dass kumulierte Wärmeäquivalente betrachtet werden, wie beispielsweise auch im NSF3- und/oder EN ISO 15883-Standard, welche beispielsweise eingesetzt werden können. Der Begriff des Aufsummierens und der Begriff des Kummulierens werden im Folgenden zumindest weitgehend synonym verwendet. Diese Aufsummierung kann beispielsweise über diskrete Zeitscheiben erfolgen, beispielsweise Zeitscheiben von einer Sekunde, wie dies im NSF3-Standard vorgeschlagen wird. Da die Zeitscheiben grundsätzlich beliebig klein gewählt werden können, kann diese Aufsummierung der Wärmeäquivalente im Grenzfall eine Integration der Wärmeäquivalente über die Zeit umfassen. Wie unten noch näher ausgeführt wird, kann die Aufsummierung jedoch, im Unterschied zu bekannten Verfahren, mit einer nicht notwendigerweise mathematisch funktionalen Wichtungsfunktion und/oder unter kontrollierter Geringgewichtung und/oder Entwertung bestimmter Temperaturbereich erfolgen.

Insoweit kann das genannte Verfahren also zunächst beispielsweise ganz oder teilweise den in WO 2006/097294 A1 oder in DE 10 2007 021 245 A1 beschriebenen Verfahren entsprechen, bei welchen ebenfalls Wärmeäquivalente kumuliert werden. Zur Erfassung der Temperaturen werden in der Reinigungsvorrichtung ein oder mehrere Temperatursensoren vorgesehen, welche beispielsweise die Temperatur unmittelbar am Reinigungsgut und/oder an anderen Stellen der Reinigungsvorrichtung erfassen, beispielsweise in einem oder mehreren Tanks, zur Bereitstellung von Reinigungsfluid. Beispielsweise können die Temperatursensoren ein oder mehrere temperaturabhängige Widerstände umfassen und/oder andere Arten von Temperatursensoren, beispielsweise infrarotoptische Temperatursensoren. Besonders bevorzugt ist die Erfassung der Temperatur unmittelbar an dem Reinigungsgut. Der eine oder die mehreren Temperatursensoren können beispielsweise mit der Steuerung der Reinigungsvorrichtung verbunden sein und/oder ganz oder teilweise. Bestandteil dieser Steuerung sein, so dass die Temperatursignale beispielsweise unmittelbar an die Steuerung geliefert werden können. Dabei kann beispielsweise auch eine Vorverarbeitung der Temperatursignale erfolgen, so dass diese beispielsweise gefiltert werden und/oder bereits zumindest teilweise in Wärmeäquivalente umgewandelt werden.

Zusätzlich wird erfindungsgemäß jedoch vorgeschlagen, auch eine Kompensation von Abweichungen bekannter Keimabtötungsstandards bzw. Desinfektionsstandards zu ermöglichen, beispielsweise Abweichungen aufgrund der oben beschriebenen "lag of regrowth-Effekte". Auch andere, beispielsweise Zielkeim-spezifische Effekte, können dabei berücksichtigt werden.

Dementsprechend wird erfindungsgemäß vorgeschlagen, die Wärmeäquivalente zusätzlich mit mindestens einer Kompensationsfunktion zu wichten. Unter einer derartigen Wichtung kann beispielsweise die Umwandlung der Wärmeäquivalente in Funktionswerte der Kompensationsfunktion verstanden werden und/oder, was im Rahmen der vorliegenden Erfindung bevorzugt ist, eine einfache Multiplikation der Wärmeäquivalente mit Kompensationswerten. Die Kompensationsfunktion ist dabei eine Funktion der Temperatur, bei welcher die Wärmeäquivalente erfasst wurden, und ist eingerichtet, um in mindestens einem Ausnahmetemperaturbereich erfasste Wärmeäquivalente mit einer geringeren Wichtung zu versehen als in anderen Temperaturbereichen erfasste Wärmeäquivalente. Die Kompensationsfunktion muss dabei nicht notwendigerweise eine Funktion im mathematischen Sinne sein, sondern kann beispielsweise auch eine Zuordnung, beispielsweise eine Relation und/oder eine Liste und/oder eine Tabelle, von Kompensationswerten als Wichtungsfaktoren umfassen. Dies wird unten noch näher ausgeführt.

Die Temperatur wird somit im Rahmen des erfindungsgemäßen Verfahrens doppelt verwendet, nämlich einerseits zur Berechnung der Wärmeäquivalente und andererseits zur zusätzlichen Wichtung der Wärmeäquivalente zur Kompensation beispielsweise von "lag of regrowth-Effekten". Der Ausnahmetemperaturbereich kann beispielsweise ein oder mehrere offene und/oder halboffene und/oder geschlossene Temperaturintervalle umfassen. Beispielsweise kann es sich dabei um Temperaturintervalle handeln, innerhalb derer zwar zunächst eine erfolgreiche Keimabtötung erfolgt, bei denen es jedoch später zu unerwünschten Effekten kommen kann, beispielsweise den oben beschriebenen "lag of regrowth-Effekten". In derartigen Ausnahmetemperaturbereichen, welche beispielsweise experimentell ermittelt werden können, beispielsweise für einen oder mehrere von einem Benutzer und/oder einem anderen Computer oder Computersystem vorgegebene Zielkeime, kann eine geringere Wichtung der Wärmeäquivalente erfolgen als in anderen Temperaturbereichen, beispielsweise in Temperaturbereichen, in welchen, wie experimentell ermittelt werden kann, "lag of regrowth-Effekte" nicht oder lediglich in geringerem Maße auftreten.

Die Kompensationsfunktion kann insbesondere derart eingerichtet sein, dass Wärmeäquivalente, die in dem mindestens einen Ausnahmetemperaturbereich erfasst wurden, bei der Summation nicht berücksichtigt werden. Dies kann beispielsweise dadurch erfolgen, dass die Wärmeäquivalente in diesem Ausnahmetemperaturbereich mit einem Faktor 0 multipliziert werden. Wärmeäquivalente außerhalb dieses Ausnahmetemperaturbereichs, also Wärmeäquivalente, die in anderen Temperaturbereichen erfasst wurden, können hingegen beispielsweise mit Faktoren größer als Null multipliziert werden, beispielsweise einem Faktor Eins. Dementsprechend kann die Kompensationsfunktion beispielsweise eine einfache digitale Funktion umfassen. So kann die Kompensationsfunktion derart eingerichtet sein, dass die Kompensationsfunktion bzw. der Funktionswert dieser Kompensationsfunktion für Temperaturen innerhalb des Ausnahmebereichs einen Wert kleiner als Eins einnimmt, insbesondere einen Wert zwischen Null und Eins, insbesondere den Wert Null, und für Temperaturen außerhalb des Ausnahmetemperaturbereichs den Wert 1. Auf diese Weise kann insbesondere sichergestellt werden, dass ein vorgegebener Desinfektionsgrad, beispielsweise ein vorgegebener kumulierter Wert an Wärmeäquivalenten, bei dem erfindungsgemäßen Verfahren mindestens erreicht wird. Das erfindungsgemäße Verfahren kann also derart durchgeführt werden, dass diese in jedem Fall gängigen Normen genügen kann, beispielsweise den oben genannten Normen, so dass beispielsweise mindestens ein vorgegebener $A_0$-Wert erreicht wird. Insofern ist, ohne dass hierdurch gegen vorgegebene Normen verstoßen würde, das vorgeschlagene Verfahren ohne größere Anpassungen auch in bestehende Reinigungsvorrichtungen integrierbar.

Der mindestens eine Ausnahmetemperaturbereich bzw. die Kompensationsfunktion können dabei auf unterschiedliche Weisen ausgestaltet sein. Eine Möglichkeit besteht beispielsweise darin, dass der mindestens eine Ausnahmetemperaturbereich oder, wenn mehrere Ausnahmetemperaturbereiche vorgesehen sind, einer dieser Ausnahmetemperaturbereiche am unteren Ende des Temperaturbereichs angeordnet ist, ab welchem Wärmeäquivalente gezählt werden. So kann sich der Ausnahmetemperaturbereich bzw. einer der Ausnahmetemperaturbereiche beispielsweise zwischen einer Mindesttemperatur eines für die Erfassung der Wärmeäquivalente verwendeten Standards und mindestens einer vorgegebenen Regrowth-Temperatur erstrecken. Die Mindesttemperatur kann beispielsweise eine Temperatur sein, ab welcher Wärmeäquivalente gezählt werden, beispielsweise 62 °C (entsprechend 143 °F) für den NSF3-Standard oder 65 °C für den EN ISO 15883-Standard. Die Regrowth-Temperatur kann beispielsweise eine beliebige Temperatur sein, welche größer gewählt wird als die Mindesttemperatur. Vorzugsweise kann die Regrowth-Temperatur derart gewählt werden, dass ein verzögertes Nachwachsen scheinbar abgetöteter Testkeime bzw. Zielkeime und/oder nur teilweise abgetöteter Testkeime bzw. Zielkeime und/oder zumindest temporär inaktivierter Testkeime bzw. Zielkeime zumindest vermindert erfolgt. Unter einem verminderten Nachwachsen kann insbesondere ein zeitverzögertes Nachwachsen, insbesondere ein stark zeitverzögertes Nachwachsen verstanden werden, beispielsweise mit einer Zeitverzögerung um mehrere Wochen oder in stark vermindertem Maße.

Bei dieser Verfahrensvariante kann beispielsweise die Zählung von Wärmeäquivalenten erst ab oder erst oberhalb einer bestimmten Regrowth-Temperatur aktiviert werden, so dass beispielsweise die Zählung von $A_0$-Werten erst oberhalb dieser Regrowth-Temperatur erfolgt. Somit werden alle normalerweise gezählten Wärmeäquivalente im Bereich zwischen der Mindesttemperatur bis zu der definierten Regrowth-Temperatur typischerweise unberücksichtigt belassen.

Die Regrowth-Temperatur wird grundsätzlich oberhalb der Mindesttemperatur gewählt und abhängig gemacht von einem oder mehreren Testkeimen und/oder vorgegebenen Zielkeimen. Beispielsweise können die im NSF3-Standard und/oder die im EN ISO 15883-Standard verwendeten Testkeime verwendet werden. Alternativ kann es jedoch beispielsweise auch einem Benutzer ermöglicht werden, einen oder mehrere Zielkeime einzugeben, welchem, beispielsweise automatisch, eine Regrowth-Temperatur zugeordnet wird, beispielsweise indem aus einer Liste von Regrowth-Temperaturen eine dem Zielkeim zugeordnete Regrowth-Temperatur ausgewählt wird. Beispielsweise kann diese Liste eine elektronische Tabelle umfassen, welche in der Steuerung hinterlegt sein kann. Der Zielkeim kann beispielsweise, wie unten noch näher erläutert wird, einen Erreger umfassen, mit welchem das Reinigungsgut mit hoher Wahrscheinlichkeit kontaminiert ist.

Die mindestens eine Regrowth-Temperatur kann beispielsweise durch entsprechende Experimente ermittelt werden und für eine Vielzahl von Zielkeimen beispielsweise hinterlegt werden. Beispielsweise können entsprechende Regrowth-Experimente mit einer Vielzahl von Zielkeimen vorgenommen werden und die zugehörigen Regrowth-Temperaturen können derart bestimmt werden, dass bei diesen Regrowth-Temperaturen das Nachwachsen in stark vermindertem Umfang oder überhaupt nicht mehr erfolgt. Die Regrowth-Temperatur kann jedoch auch durch entsprechende Erwartungswerte oder Vermutungen gewählt werden. Beispielsweise kann die Regrowth-Temperatur in einem Temperaturintervall zwischen 67 °C und 75 °C, vorzugsweise in einem Temperaturintervall zwischen 70 °C und 87 °C, und besonders bevorzugt in einem Temperaturintervall zwischen 75 °C und 85 °C gewählt werden.

Alternativ oder zusätzlich zu der oben beschriebenen Verfahrensvariante, bei welcher der Ausnahmetemperaturbereich am unteren Ende der Temperaturskala, ab welcher Wärmeäquivalente gezählt werden, gewählt wird, lässt sich der Ausnahmetemperaturbereich bzw., wenn mehrere Ausnahmetemperaturbereiche vorgegeben werden, einer oder mehrere dieser Ausnahmetemperaturbereiche auf andere Weise wählen. So kann der Ausnahmetemperaturbereich bzw. einer der Ausnahmetemperaturbereiche oder mehrere der Ausnahmetemperaturbereiche auch derart gewählt werden, dass zwischen der Mindesttemperatur eines für die Erfassung der Wärmeäquivalente verwendeten Standards (beispielsweise wiederum NSF3-Standard und/oder EN ISO 15883-Standard) und der Anfangstemperatur des Ausnahmetemperaturbereichs mindestens ein nicht dem Ausnahmetemperaturbereich zugeordneter Temperaturbereich angeordnet ist. In anderen Worten kann der Ausnahmetemperaturbereich derart gewählt werden, dass die Zählung der Wärmeäquivalente für einen bestimmten Ausnahmetemperaturbereich unterbrochen wird. Beispielsweise kann die Zählung von $A_0$-Werten in dem Ausnahmetemperaturbereich unterbrochen werden, so dass die normalerweise in diesem Bereich gezählten Wärmeäquivalente für die kumulierten Wärmeäquivalente unberücksichtigt bleiben.

Wie oben dargestellt, kann durch die kumulierten Wärmeäquivalente insbesondere eine Mindest-Desinfektionswirkung erreicht werden. Diese Mindest-Desinfektionswirkung kann beispielsweise eine Zieldesinfektionswirkung umfassen, wobei die Zieldesinfektionswirkung eine Mindestsumme an Wärmeäquivalenten umfasst. Das Verfahren kann dann beispielsweise mindestens so lange durchgeführt werden, bis die Summation der Wärmeäquivalente die genannte Mindestsumme ergibt. Beispielsweise kann die Zieldesinfektionswirkung durch einen Benutzer und/oder einen anderen Computer oder ein anderes Computernetzwerk vorgegeben werden, beispielsweise über die oben beschriebene Schnittstelle.

Neben der oben genannten Temperatur können weitere Größen erfasst werden, beispielsweise von der Reinigungsvorrichtung und/oder der Steuerung der Reinigungsvorrichtung, welche bei der Auswahl der Kompensationsfunktion ebenfalls berücksichtigt werden können. So können beispielsweise auch andere Einflussgrößen, zusätzlich zur Temperatur, bei welcher die Wärmeäquivalente erfasst werden, einen Einfluss auf die Desinfektionswirkung, insbesondere die Langzeit-Desinfektionswirkung, haben. Eine besondere Rolle kann insbesondere ein Gradient der Temperatur spielen, insbesondere ein zeitlicher Gradient und/oder ein räumlicher Gradient. So kann beispielsweise ein "lag of regrowth-Effekt" von einem zeitlichen und/oder räumlichen Gradienten der Temperatur abhängig sein, insbesondere einem zeitlichen Gradienten, beispielsweise einer schnellen Temperaturänderung im Vergleich zu einer langsamen Temperaturänderung. Beispielsweise kann bei zunehmendem Gradienten der Regrowth-Effekt verstärkt auftreten. Dementsprechend kann beispielsweise die Kompensationsfunktion ebenfalls an diesen Gradienten angepasst werden. Beispielsweise können bei zunehmendem Gradienten niedrige Temperaturen in verstärktem Maße unberücksichtigt bleiben, so dass beispielsweise bei einem oder beiden der oben beschriebenen Verfahrensvarianten die Regrowth-Temperatur mit zunehmendem Gradient der Temperatur nach oben gesetzt werden kann. Dies kann beispielsweise wiederum durch eine automatische Zuordnung erfolgen, beispielsweise indem dem Gradienten mittels einer automatischen Auswahl, beispielsweise aus einer elektronischen Tabelle, die Regrowth-Temperatur zugeordnet wird. Die Regrowth-Temperatur kann als also von einer oder mehreren Größen abhängig sein, beispielsweise mindestens einem Zielkeim und/oder mindestens einem Gradienten und/oder weiteren erfassten Messgrößen. Beispielsweise kann eine Zahl von Kompensationsfunktionen vorgegeben werden, wobei eine automatische Zuordnung entsprechend der erfassten Größen erfolgt, beispielsweise in der Steuerung. Alternativ oder zusätzlich kann, wie oben beschrieben, die Zuordnung bzw. Auswahl der mindestens einen Kompensationsfunktion auch unter Berücksichtigung mindestens einer Information über mindestens einen Zielkeim erfolgen, mit welchem das Reinigungsgut verunreinigt sein könnte.

Die Reinigungsvorrichtung kann entsprechende Vorrichtungen umfassen, um das Verfahren in einer oder mehreren der oben beschriebenen Varianten durchzuführen. Die Reinigungsvorrichtung ist eingerichtet, um einem Benutzer und/oder einer externen Datenverarbeitungsvorrichtung eine Eingabe einer oder mehrerer Größen zu ermöglichen. Beispielsweise können dies zusätzlich zu eines Kompensationsfunktion ein Ausnahmetemperaturbereich und/oder ein Zielkeim, mit welchem das Reinigungsgut kontaminiert sein könnte, und/oder eine zu erwartende Lagerdauer des Reinigungsguts nach der Reinigung und/oder Kombinationen der genannten und/oder anderer Größen sein. Wie oben dargestellt, kann die Eingabe beispielsweise über eine Eingabe/Ausgabe-Schnittstelle erfolgen. Die zu erwartende Lagerdauer kann beispielsweise dahingehend berücksichtigt werden, dass Reinigungsgut, welches bis zur nächsten Benutzung ohnehin nur für eine relativ kurze Zeitdauer gelagert wird, mit einer niedrigeren Regrowth-Temperatur behandelt wird. Beispielsweise können "lag of regrowth-Effekte", bei denen ein Wiederanwachsen der Vitalität der Zielkeime erst lange nach einer zu erwartenden erneuten Benutzung eintreten könnte, in Kauf genommen werden, so dass beispielsweise eine niedrige Regrowth-Temperatur gewählt wird. Ist jedoch eine längere Lagerung des Reinigungsguts geplant, so können beispielsweise höhere Regrowth-Temperaturen vorgesehen sein.

Das vorgeschlagene Verfahren und die vorgeschlagene Reinigungsvorrichtung weisen gegenüber bekannten Verfahren und Vorrichtungen eine Vielzahl von Vorteilen auf. Im Gegensatz zu bekannten starren Verfahren und Vorrichtungen, welche beispielsweise exakt nach vorgegebenen Hygienestandards arbeiten, können das vorgeschlagene Verfahren und die vorgeschlagene Reinigungsvorrichtung flexibel auf die tatsächlichen Gegebenheiten reagieren und über eine Mindesthygienewirkung hinaus auch Langzeit-Hygienewirkungen sicherstellen. Einflussgrößen, wie beispielsweise die Temperatur und/oder der Temperaturgradient und/oder weitere Größen, können beispielsweise als Echtzeitwerte erfasst werden, um gegebenenfalls in die Kompensationsfunktion einbezogen zu werden, beispielsweise in eine Berechnung und/oder Filterung. Der Einfluss derartiger zusätzlicher Parameter ist beispielsweise aus dem Bereich der Pasteurisierung bekannt. Die Kompensationsfunktion in der Reinigungsvorrichtung kann beispielsweise derart ausgestaltet sein, dass die Korrektur umso größer ist, je größer der Gradient der Temperatur ausgestaltet ist. Beispielsweise kann eine Software der Steuerung eingerichtet sein, um derartige Einflussgrößen zu handhaben. Da eine flexible Eingabe der Kompensationsfunktion möglich ist, kann auch ein regelmäßiges und/oder unregelmäßiges Update der Steuerung erfolgen, beispielsweise wenn weitere wissenschaftliche Erkenntnisse vorliegen. Auch eine Implementierung von nach Mikroorganismenklassen differenzierten Parametersätzen kann erfolgen, beispielsweise über die Eingabe eines oder mehrerer der oben beschriebenen Zielkeime. Das vorgeschlagene Verfahren kann auf einfache Weise auch in bereits vorhandenen Steuerungen implementiert werden, indem diese entsprechend programmtechnisch eingerichtet werden. Das Verfahren kann dabei beispielsweise als Option zu einer bereits vorhandenen Software implementiert werden und/ oder als Standard festgelegt werden. Gleichzeitig kann jedoch eine vorgegebene Mindest-Hygienisierung sichergestellt werden, da die oben beschriebene Kompensation beispielsweise derart erfolgen kann, dass das Reinigungsgut mindestens nach einem vorgegebenen Standard hygienisiert wird. Auf diese Weise können beispielsweise eine Hygienesicherheit und ein Desinfektionsniveau im Vergleich zur herkömmlichen $A_0$-Wert-Methode sichergestellt oder sogar gesteigert werden, da beispielsweise lediglich normalerweise mitgezählte Thermoäquivalente durch die Kompensationsfunktion bei der Kumulierung ausgelassen werden können. Die Modifizierung bekannter Reinigungsvorrichtungen kann somit derart eingerichtet sein, dass diese nach wie vor anerkannten Standards genügt.

Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigen:

Figur 1     ein Ausführungsbeispiel einer erfindungsgemäßen Reinigungsvorrichtung;

Figur 2A    ein schematisches Ablaufdiagramm eines Verfahrens gemäß dem Stand der Technik;

Figur 2B    ein schematisches Ablaufdiagramm eines erfindungsgemäßen Verfahrens;

Figur 3     eine Aufheizkurve und kumulierte Wärmeäquivalente nach einem her- kömmlichen Verfahren;

Figur 4A    eine Kompensationsfunktion nach einem ersten Ausführungsbeispiel eines erfindungsgemäßen Verfahrens;

Figur 4B    ein kumuliertes Wärmeäquivalent nach der ersten Ausführungsform des erfindungsgemäßen Verfahrens;

Figur 5A    eine Kompensationsfunktion gemäß einem zweiten Ausführungsbeispiel des erfindungsgemäßen Verfahrens; und

Figur 5B    kumulierte Wärmeäquivalente gemäß dem zweiten Ausführungsbeispiel des erfindungsgemäßen Verfahrens.

Ausführungsbeispiele

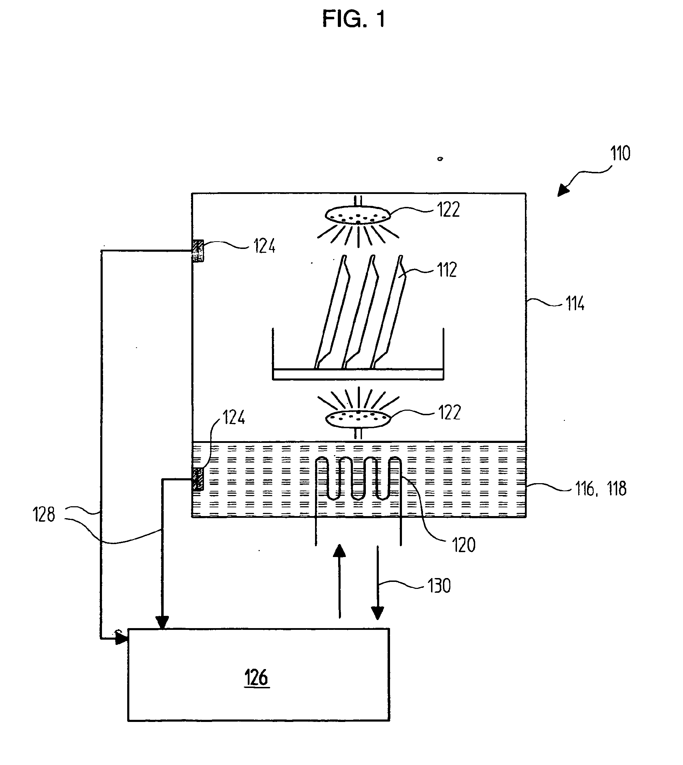

In Figur 1 ist in einer stark vereinfachten Form ein Ausführungsbeispiel einer erfindungsgemäßen Reinigungsvorrichtung 110 in schematisierter Darstellung gezeigt. Die Reinigungsvorrichtung 110 dient zur Reinigung von stilisiert angedeutetem Reinigungsgut 112 in einer Reinigungskammer 114 durch Verwendung eines Reinigungsfluids 116. Beispielsweise kann es sich bei diesem Reinigungsgut 112 um Steckbecken, Bettpfannen, Urinflaschen, Nachtgeschirr oder ähnliches Reinigungsgut handeln. Auch anderer Arten von Reinigungsgut 112 sind jedoch grundsätzlich verwendbar. Das Reinigungsfluid 116 ist teilweise in einem Tank 118 aufgenommen und mittels einer Heizvorrichtung 120 beheizt. Das Reinigungsfluid 116 wird mittels eines Düsensystems 122 auf das Reinigungsgut 112 aufgesprüht. Die Temperatur des Reinigungsguts 112 und/oder des Reinigungsfluids 116 wird mittels eines oder mehrerer Temperatursensoren 124 überwacht.

In der dargestellten Reinigungsvorrichtung 110 ist das Reinigungsfluid 116 als flüssiges Reinigungsfluid angedeutet. Alternativ oder zusätzlich kann jedoch auch ein gasförmiges Reinigungsfluid 116 eingesetzt werden, beispielsweise Heißdampf zur Hygienisierung des Reinigungsguts 112. In diesem Fall kann der mindestens eine Temperatursensor 124 beispielsweise auch eine Dampftemperatur im Inneren der Reinigungskammer 114 überwachen. Alternativ oder zusätzlich kann der Reinigungssensor 124 auch beispielsweise mittels einer Infrarotmessung eine Temperatur unmittelbar auf dem Reinigungsgut 112 überwachen. Verschiedene Ausführungsformen sind möglich und aus dem Stand der Technik bekannt.

Weiterhin weist die Reinigungsvorrichtung 110 eine Steuerung 126 auf, welche beispielsweise über Signalleitungen 128 Signale der Temperatursensoren 124 abfragen kann und/oder über mindestens eine Schnittstelle 130 weitere Komponenten der Reinigungsvorrichtung 110 steuern und/oder regeln kann.

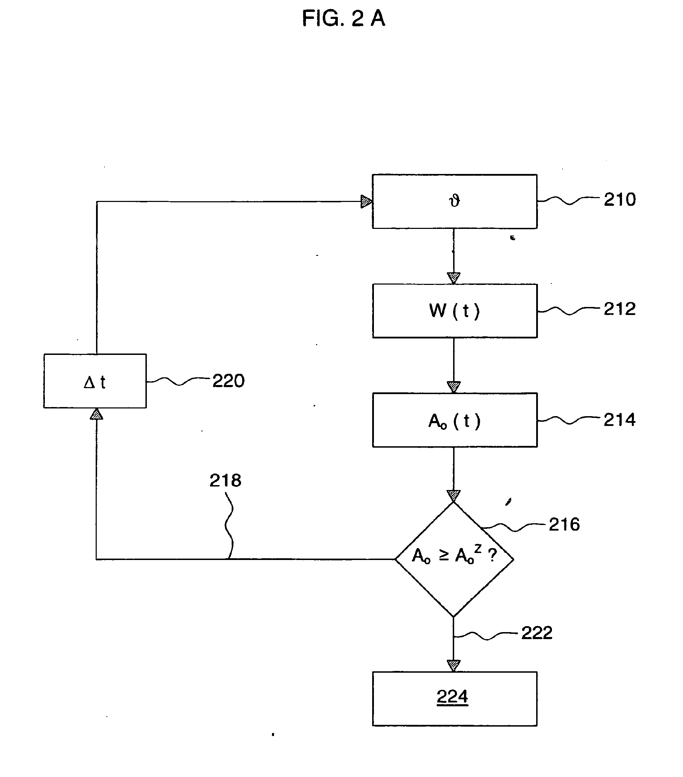

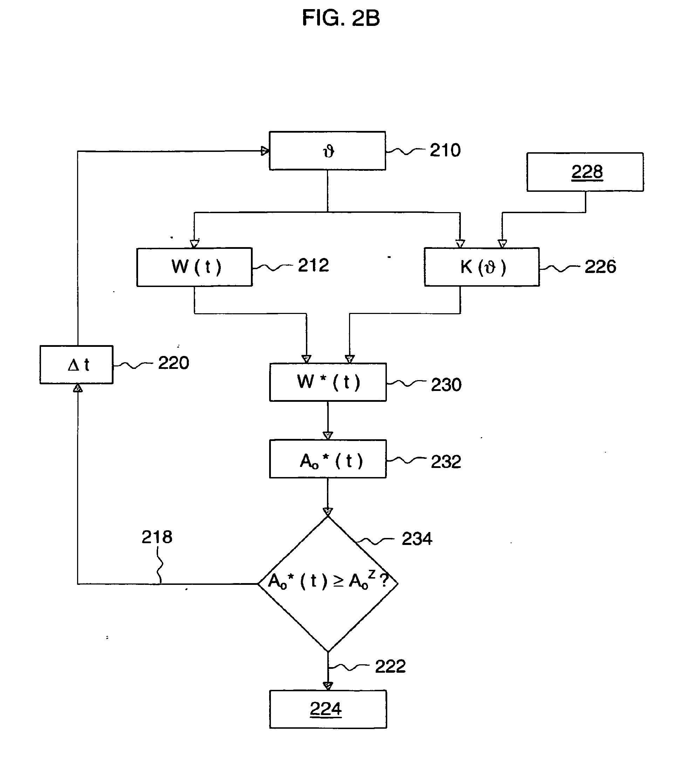

In den Figuren 2A und 2B sind Ablaufdiagramme von Verfahren zur Reinigung von Reinigungsgut unter Sicherstellung einer thermischen Hygienewirkung dargestellt. Dabei zeigt Figur 2A ein Ablaufdiagramm eines dem Stand der Technik entsprechenden Verfahrens, wohingegen Figur 2B eine erfindungsgemäße Modifikation des Verfahrens gemäß Figur 2A zeigt. Exemplarisch sei im Folgenden angenommen, dass das Verfahren gemäß Figur 2A der Norm EN ISO 15883 folgt, so dass beispielsweise auf die in Anhang A dieser Norm gegebenen Definitionen verwiesen werden kann. Auch andere Normen sind jedoch alternativ oder zusätzlich anwendbar, beispielsweise die oben zitierte NSF3-Norm.

Zunächst soll das in Figur 2A aufgeführte, dem Stand der Technik entsprechende Verfahren beschrieben werden. Dieses Verfahren kann beispielsweise in der Steuerung 126 einer Reinigungsvorrichtung 110 gemäß Figur 1 implementiert werden.

In einem ersten Verfahrensschritt (Schritt 210) wird eine Temperatur $\vartheta$ erfasst. Aus dieser Temperatur $\vartheta$ wird in einem anschließenden Verfahrensschritt 212 eine Größe berechnet, welche hier und im Folgenden allgemein als Wärmeäquivalent bezeichnet wird und welche allgemein den Funktionswert einer monoton wachsenden Funktion, angewandt auf die Temperatur $\vartheta$, darstellt. In der Norm EN ISO 15883 bestimmt sich dieses Wärmeäquivalent, welches im Folgenden mit dem Buchstaben W bezeichnet wird, zu einem Zeitpunkt t nach der folgenden Formel:

$$W(t) = 10^{(\vartheta-80)/z} \qquad (1).$$

Dabei wird die Temperatur $\vartheta$ in °C angegeben, und es werden lediglich Temperaturen $\vartheta$ von mindestens 65 °C berücksichtigt. Der Wert z (z-Wert) ist nach EN ISO 15883 die Temperaturänderung in °K, welche erforderlich ist, um in einem Desinfektionsprozess mit feuchter Hitze eine 10-fache Änderung der mikrobiellen Inaktivierungsrate zu erzielen. Dieser z-Wert kann abhängig sein von bestimmten Keimen, beispielsweise dem im Verfahren vorgegebenen Zielkeimen.

In einem weiteren Verfahrensschritt 214 werden die Wärmeäquivalente über die Zeit kumuliert. Dies kann beispielsweise kontinuierlich durch Integration erfolgen oder, wie in EN ISO 15883, über diskrete Zeitscheiben, welche in EN ISO 15883 zu $\Delta t = 1s$ festgelegt sind. Auch andere Zeitintervalle und/oder Zeitintervalle mit unterschiedlicher Länge sind jedoch verwendbar. Nach EN ISO 15883 erfolgt diese Kumulierung zum sogenannten $A_0$-Wert nach der folgenden Formel:

$$A_0 = \sum_t W(t) \cdot \Delta t \qquad (2).$$

In Verfahrensschritt 216 wird abgefragt, ob die kumulierten Wärmeäquivalente eine Mindeststumme erreicht haben, welche hier mit bezeichnet ist. Ist dies nicht der Fall (Zweig 218 in Figur 2A), so wird nach einer Wartezeit von $\Delta t$ (Schritt 220 in Figur 2A) die Messung wiederholt, so dass die Verfahrensschritte 210 bis 216 erneut durchgeführt werden. Ist hingegen die Mindestsumme erreicht (Zweig 222 in Figur 2A), so kann das Programm beendet werden (Schritt 224). Somit ist nach diesem konventionellen Verfahren sichergestellt, dass das Reinigungsgut 112 mit einer Mindestsumme an Wärmeäquivalenten beaufschlagt wird.

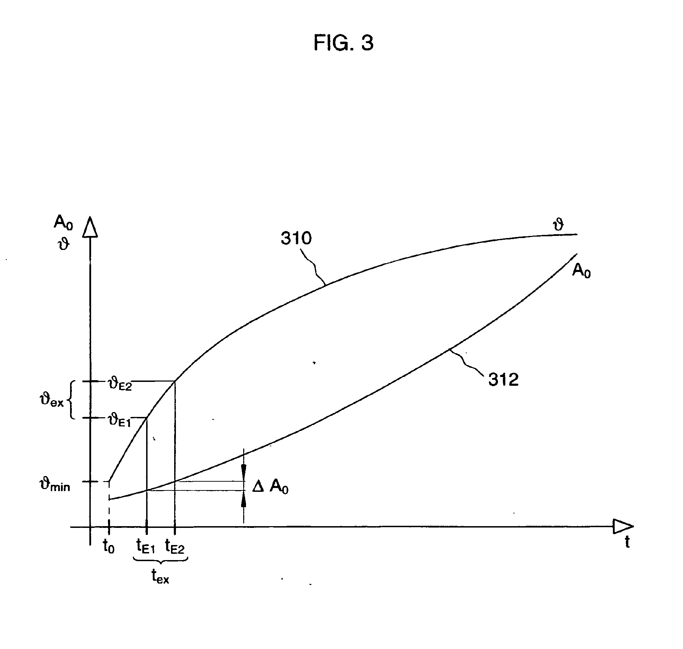

In Figur 3 ist eine typische Aufheizkurve (Kurve 310, entsprechend der Achsenbeschriftung $\vartheta$) von Reinigungsgut 112 als Funktion der Zeit t aufgetragen. Weiterhin ist eine zeitliche Entwicklung der kumulierten Wärmeäquivalente in einer Kurve 312 aufgetragen (Achsenbeschriftung $A_0$).

Aus der Darstellung ist erkennbar, dass das Reinigungsgut 112 zu einem Zeitpunkt $t_0$ eine Mindesttemperatur $\vartheta_{min}$ erreicht, ab welcher nach dem jeweiligen Standard die Wärmeäquivalente gezählt werden. Bei EN ISO 15883 ist dies beispielsweise eine Temperatur von 65 °C. Zu diesem Zeitpunkt nimmt die $A_0$-Kurve 312 den Wert 0 ein und wächst von dort an monoton an. So steigert sich der $A_0$-Wert beispielsweise in einem Intervall zwischen $\vartheta_{E1}$ und $\vartheta_{E2}$ um einen

Betrag $\Delta A_0$.

Das in Figur 2A gezeigte Verfahren berücksichtigt jedoch unter Umständen "lag of regrowth-Effekte" nicht. In Figur 2B ist dementsprechend eine erfindungsgemäße Modifikation des Verfahrens dargestellt, bei welcher zunächst ebenfalls wieder in Verfahrensschritt 210 während eines Reinigungsvorgangs des Reinigungsguts 112 die Temperatur $\vartheta$ bestimmt wird. Aus dieser Temperatur $\vartheta$ wird in Verfahrensschritt 212, analog zur Figur 2A, wiederum das Wärmeäquivalent W(t) bestimmt, wobei beispielsweise wiederum die oben genannte Formel (1) verwendet werden kann.

Gleichzeitig oder zeitversetzt zum Verfahrensschritt 212 wird jedoch in Verfahrensschritt 226 eine Kompensationsfunktion K bestimmt. Der Funktionswert dieser Kompensationsfunktion K ist abhängig von der Temperatur $\vartheta$. Gleichzeitig kann die Kompensationsfunktion K jedoch auch von einem oder mehreren Zielkeimen abhängig sein, mit denen das Reinigungsgut 112 mit hoher Wahrscheinlichkeit kontaminiert ist. Die Eingabe derartiger Zielkeime ist in Figur 2B mit Verfahrensschritt 228 symbolisiert.

Die Kompensationsfunktion stellt eine Wichtung der Wärmeäquivalente W(t) bereit, welche "lag of regrowth-Effekte" vermeiden soll. So können beispielsweise Temperaturbereiche, in welchen eine Desinfektion zwar zur Zählung der Wärmeäquivalente $A_0$ beiträgt, bei welchen jedoch später verstärkt "lag of regrowth-Effekte" eintreten können, gezielt geringer gewichtet werden oder sogar ganz bei der Zählung ausgeblendet werden. Dementsprechend kann die Kompensationsfunktion K eine einfache Multiplikation der Wärmeäquivalente W(t) aus Verfahrensschritt 212 mit der Kompensationsfunktion K beinhalten. Auch komplexere Kompensationsfunktionen, welche auf W(t) einwirken, sind jedoch möglich. Bei einer einfachen Multiplikation kann beispielsweise, wie in Figur 2B angedeutet, in Verfahrensschritt 230 eine Wichtung des Wärmeäquivalents W(t) zu einem gewichteten Wärmeäquivalent W$^*$(t) erfolgen, beispielsweise nach der folgenden Formel:

$$W^*(t, \vartheta) = K(\vartheta) \cdot W(t) \qquad (3).$$

In Verfahrensschritt 232 werden die gewichteten Wärmeäquivalente W$^*$(t) aus Verfahrensschritt 230 kumuliert, analog zu Verfahrensschritt 214 in Figur 2A. Dies erfolgt beispielsweise nach der folgenden Formel:

$$A_0^* = \sum_t W^*(t) \cdot \Delta t \qquad (4).$$

Anschließen erfolgt in Verfahrensschritt 234, analog zu Verfahrensschritt 216 in Figur 2A, eine Abfrage, ob die kumulierten, gewichteten Wärmeäquivalente bereits die Mindestsumme erreicht haben. Ist dies nicht der Fall (Zweig 218), so kann nach einer Wartezeit $\Delta t$ (Schritt 220) das Verfahren mit der Messung und der Kumulierung wiederholt werden. Dabei müssen nicht notwendigerweise alle Verfahrensschritte wiederholt werden. Nicht notwendigerweise wiederholt werden muss beispielsweise die ohnehin optionale Eingabe eines Zielkeims in Schritt 228, sondern es wird lediglich ein Funktionswert K($\vartheta$) der neu gemessenen Temperatur $\vartheta$ in Schritt 226 bestimmt. Ist hingegen die Mindestsumme erreicht (Zweig 222 in Figur 2B), so kann das Verfahren grundsätzlich beendet werden.

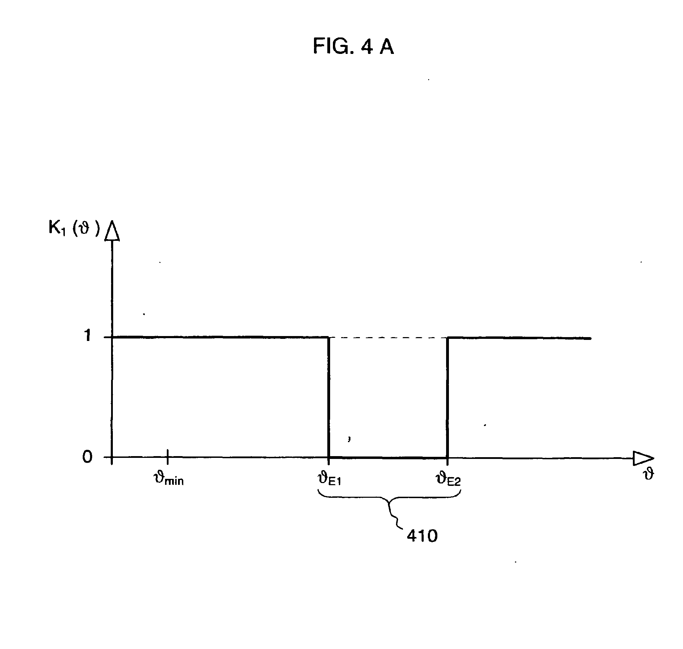

Die Kompensationsfunktion K($\vartheta$) kann grundsätzlich, wie oben dargestellt, Temperaturbereiche ausblenden, welche zwar nach herkömmlichen Verfahren zu den kumulierten Wärmeäquivalenten $A_0$ beitragen, welche jedoch hinsichtlich des "lag of regrowth-Effekts" zu einer ineffizienteren Hygienisierung führen. In Figur 4A ist ein erstes Ausführungsbeispiel einer Kompensationsfunktion K$_1$($\vartheta$) als Funktion der Temperatur $\vartheta$ des Reinigungsguts 112 dargestellt, bei welcher ein Ausnahmetemperaturbereich (in Figur 4A symbolisch mit der Bezugsziffer 410 bezeichnet) bei der Berechnung der kumulierten Wärmeäquivalente nicht berücksichtigt wird. Dieser Ausnahmetemperaturbereich erstreckt sich zwischen den Temperaturen $\vartheta_{E1}$ und $\vartheta_{E2}$, welche beide oberhalb der Minimaltemperatur $\vartheta_{min}$ liegen. Beispielsweise könnte $\vartheta_{E1}$ zwischen 65 °C und 75 °C gewählt werden und $\vartheta_{E2}$ zwischen 70 °C und 80 °C.

Mathematisch lässt sich die Kompensationsfunktion K$_1$ derart beschreiben, dass diese für die Werte außerhalb des Ausnahmetemperaturbereichs 410 den Wert 1 annimmt und für Werte innerhalb des Ausnahmetemperaturbereichs den Wert 0:

$$K_1(\vartheta) = 0 \;\; \text{für} \;\; \vartheta_{E1} \leq \vartheta \leq \vartheta_{E2} \qquad (5a)$$

$$K_1(\vartheta) = 1 \quad \text{sonst.} \qquad\qquad (5b).$$

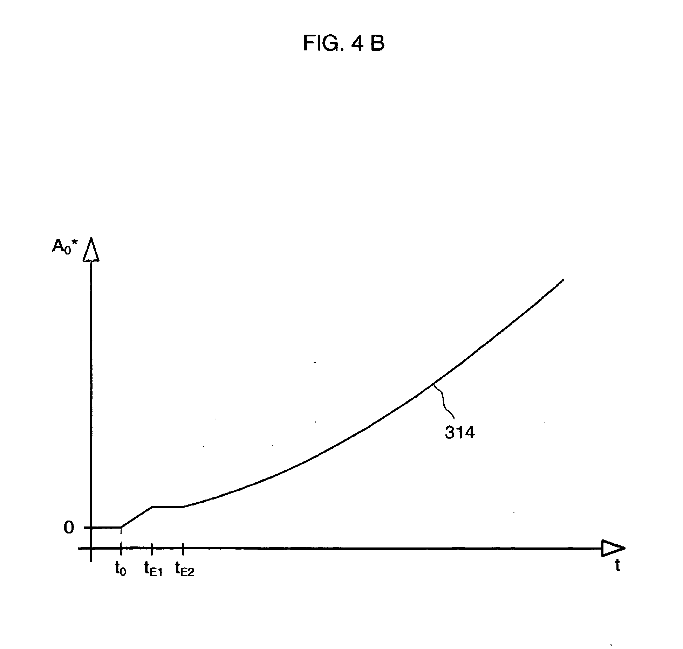

Ausgehend von dieser Kompensationsfunktion $K_1$ ist in Figur 4B das kumulierte, gewichtete Wärmeäquivalent $A_0^*$, berechnet nach Gleichung (4), als Funktion der Zeit t dargestellt. Ausgangspunkt stellt wieder die Aufheizkurve 310 in Figur 3 dar, welche zum Zeitpunkt $t_0$ die Minimaltemperatur $\vartheta_{min}$ erreicht und zu den Zeiten $t_{E1}$ bzw. $t_{E2}$ die Temperaturen $\vartheta_{E1}$ bzw. $\vartheta_{E2}$. Die Aufheizkurve in Figur 4B ist dabei, wie auch die Aufheizkurve in Figur 3, als kontinuierliche Kurve dargestellt, obwohl diese Kurve theoretisch bei Zeitscheiben von 1s eine Sprungfunktion darstellt. Im Grenzfall unendlich kleiner Zeitscheiben $\Delta t$ (also einer Integration über die Zeit) wird diese Kurve jedoch beispielsweise kontinuierlich.

Wie aus der Darstellung in Figur 4B zu erkennen ist, ändert sich während des Zeitfensters zwischen $t_{E1}$ und $t_{E2}$, also während die Temperatur des Reinigungsguts 112 innerhalb des Ausnahmetemperaturbereichs 410 in Figur 4A liegt, das kumulierte, gewichtete Wärmeäquivalent $A_0^{\overset{-}{*}}$ nicht. Erst bei Überschreiten der oberen Grenze des $\vartheta_{E2}$ des Ausnahmetemperaturbereichs 110 steigt die kumulierte, gewichtete Wärmeäquivalent-Kurve wieder an.

Auch andere Ausgestaltungen der Kompensationskurve $K(\vartheta)$ sind jedoch möglich, wie oben dargestellt wurde. So kann diese Kompensationsfunktion auch ein Funktional der Funktion W(t) sein.

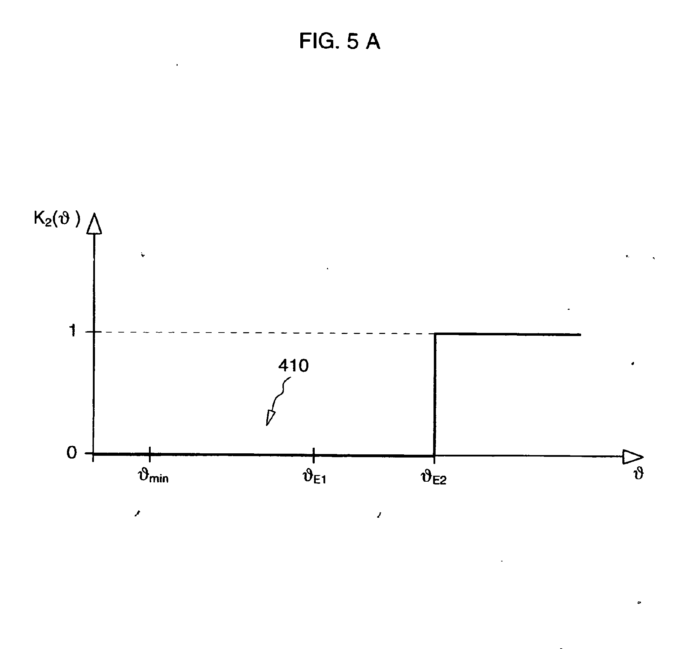

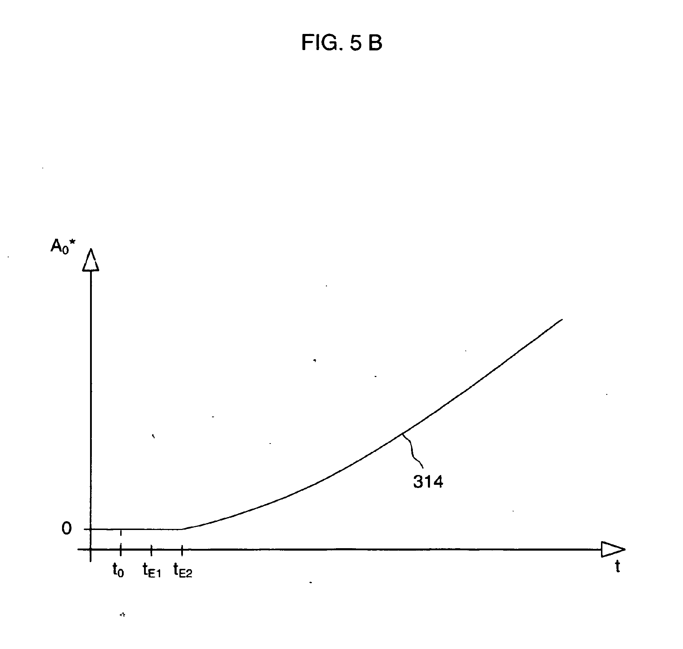

In den Figuren 5A und 5B ist ein zweites Ausführungsbeispiel eines erfindungsgemäßen Verfahrens in zu den Figuren 4A und 4B analoger Darstellung gezeigt. Dieses Beispiel stellt einen Grenzfall dar, bei welchem die untere Grenze des Ausnahmetemperaturbereichs bis hin zu $\vartheta_{min}$, also der Mindesttemperatur für den verwendeten Standard, oder sogar zu niedrigeren Temperaturen hin, verschoben ist. Physikalisch stellt dies keinen Unterschied mehr dar, da Verschiebungen der unteren Intervallgrenze des Ausnahmetemperaturbereichs 410 auf Temperaturen unterhalb von $\vartheta_{min}$ sich auf das Ergebnis nicht auswirken, da unterhalb von $\vartheta_{min}$ ohnehin keine Wärmeäquivalente kumuliert werden. Die obere Grenze des Ausnahmetemperaturbereichs 410, also die Temperatur $\vartheta_{E2}$, lässt sich auch als Regrowth-Temperatur bezeichnen, da beispielsweise diese Temperatur derart gewählt werden kann, dass ein verzögertes Nachwachsen scheinbar abgetöteter Testkeime erfolgt und/oder mindestens eines Zielkeims zumindest vermindert erfolgt. Beispielsweise kann diese Temperatur bei 80 °C oder höher liegen.

In Figur 5B ist wiederum, ausgehend von der Aufheizkurve 310 in Figur 3, eine Kurve der kumulierten, gewichteten Wärmeäquivalente $A_0^*$ für die Kompensationsfunktion $K_2$ in Figur 5A gezeigt. Wie auch in Figur 4B, ist auch hier die Kurve der kumulierten, gewichteten Wärmeäquivalente mit der Bezugsziffer 314 bezeichnet.

Die Kurve 314 in Figur 5B zeigt, dass bis zum Zeitpunkt $t_{E2}$, zu welchem die Aufheizkurve 310 in Figur 3 die Regrowth-Temperatur $\vartheta_{E2}$ erreicht, keine Wärmeäquivalente kumuliert werden. Ab diesem Zeitpunkt steigt die Kurve 314, und es werden Wärmeäquivalente kumuliert.

Bezugszeichenliste

| | |
|---|---|
| 110 | Reinigungsvorrichtung |
| 112 | Reinigungsgut |
| 114 | Reinigungskammer |
| 116 | Reinigungsfluid |
| 118 | Tank |
| 120 | Heizvorrichtung |
| 122 | Düsensystem |
| 124 | Temperatursensor |
| 126 | Steuerung |
| 128 | Signalleitungen |
| 130 | Schnittstelle |
| 210 | Erfassung Temperatur |
| 212 | Bestimmung Wärmeäquivalent |
| 214 | Kumulierung Wärmeäquivalente |
| 216 | Abfrage |
| 218 | Mindestsumme nicht erreicht |
| 220 | Wartezeit |

222    Mindestsumme erreicht
224    Ende
226    Bestimmung Kompensationsfunktion
228    Eingabe Zielkeim
230    Wichtung
232    Kumulierung gewichtete Wärmeäquivalente
310    Aufheizkurve
312    kumulierte Wärmeäquivalente
314    kumulierte, gewichtete Wärmeäquivalente
410    Ausnahmetemperaturbereich

**Patentansprüche**

1. Verfahren zur Reinigung und/oder Desinfektion eines Reinigungsguts (112), wobei das Reinigungsgut (112) mit mindestens einem Reinigungsfluid (116) beaufschlagt wird, wobei Wärmeäquivalente erfasst werden, mit welchen das Reinigungsgut (112) beaufschlagt wird, wobei die Wärmeäquivalente aufsummiert werden, **dadurch gekennzeichnet, dass** bei der Summation der Wärmeäquivalente die Wärmeäquivalente zusätzlich mit einer Kompensationsfunktion gewichtet werden, wobei die Kompensationsfunktion eine Funktion der Temperatur ist, bei welcher die Wärmeäquivalente erfasst wurden und wobei die Kompensationsfunktion in mindestens einem Ausnahmetemperaturbereich (410) erfasste Wärmeäquivalente mit einer geringeren Wichtung versieht als in anderen Temperaturbereichen erfasste Wärmeäquivalente und sich der Ausnahmetemperaturbereich (410) zwischen einer Mindesttemperatur eines für die Erfassung der Wärmeäquivalente verwendeten Standards und mindestens einer vorgegebenen Regrowth-Temperatur erstreckt, wobei die Regrowth-Temperatur größer ist als die Mindesttemperatur und die Kompensationsfunktion unter Berücksichtigung mindestens einer Information über mindestens einen Zielkeim, mit welchen das Reinigungsgut (112) verunreinigt sein könnte, gewählt wird.

2. Verfahren nach dem vorhergehenden Anspruch, wobei die Kompensationsfunktion derart eingerichtet ist, dass Wärmeäquivalente, die in dem mindestens einen Ausnahmetemperaturbereich (410) erfasst wurden, bei der Summation nicht berücksichtigt werden.

3. Verfahren nach einem der beiden vorhergehenden Ansprüche, wobei die Kompensationsfunktion für Temperaturen innerhalb des Ausnahmetemperaturbereichs (410) einen Wert kleiner als Eins annimmt, insbesondere den Wert Null, und für Temperaturen außerhalb des Ausnahmetemperaturbereichs (410) den Wert Eins.

4. Verfahren nach einem der vorhergehenden Ansprüche, wobei die Regrowth-Temperatur derart gewählt wird, dass ein verzögertes Nachwachsen scheinbar abgetöteter und/oder nur teilweise abgetöteter und/oder zumindest temporär inaktivierter Testkeime und/oder mindestens eines vorgegebenen Zielkeims zumindest vermindert erfolgt.

5. Verfahren nach einem der vorhergehenden Ansprüche, wobei die Regrowth-Temperatur in einem Temperaturintervall zwischen 67 °C und 95 °C, vorzugsweise in einem Temperaturintervall zwischen 70 °C und 87 °C und besonders bevorzugt in einem Temperaturintervall zwischen 75 °C und 85 °C gewählt wird.

6. Verfahren nach einem der vorhergehenden Ansprüche, wobei der Ausnahmetemperaturbereich (410) derart gewählt wird, dass zwischen einer Mindesttemperatur eines für die Erfasssung der Wärmeäquivalente verwendeten Standards und einer Anfangstemperatur des Ausnahmetemperaturbereichs (410) mindestens ein nicht dem Ausnahmetemperaturbereich (410) zugeordneter Temperaturbereich angeordnet ist.

7. Verfahren nach einem der vorhergehenden Ansprüche, wobei die Wärmeäquivalente nach dem NSF3-Standard oder nach dem EN ISO 15883-Standard erfasst werden.

8. Verfahren nach einem der vorhergehenden Ansprüche, wobei mindestens eine Zieldesinfektionswirkung vorgegeben wird, wobei die Zieldesinfektionswirkung eine Mindestsumme an Wärmeäquivalenten umfasst, wobei das Verfahren mindestens so lange durchgeführt wird, bis die Summation die Mindestsumme ergibt.

9. Verfahren nach einem der vorhergehenden Ansprüche, wobei weiterhin mindestens ein Gradient der Temperatur ermittelt wird.

**10.** Verfahren nach dem vorhergehenden Anspruch, wobei die Kompensationsfunktion unter Berücksichtigung des Gradienten gewählt wird.

**11.** Reinigungsvorrichtung (110) zur Reinigung und/oder Desinfektion eines Reinigungsguts (112) basierend auf der Erfassung und kumulierung von Wärmeäquivalenten, *aufgenommen in einer oder mehreren Reinigungskammern (114), mit einem Düsensystem* (122) *zur Beaufschlagung des Reinigungsguts* (112) *mit Reinigungsfluid (116) und einem oder mehreren Temperatursensoren* (124), *wobei die Reinigungsvorrichtung mindestens eine Steuerung (126) zur Steuerung des Reinigungsvorgangs umfasst mit Ein- und Ausgabemitteln für einen Benutzer und/oder eine externe Datenverarbeitungsvorrichtung, um zusätzlich zur Eingabe einer Kompensationsfunktion* zur Gewichtung der Wärmeäquivalente, *eine oder mehrere der folgenden Größen einzugeben:*

- ein Ausnahmetemperaturbereich (410)
- ein Zielkeim, mit welchem das Reinigungsgut (112) kontaminiert sein könnte
- eine zu erwartende Lagerdauer des Reinigungsguts (112) nach der Reinigung.

**12.** Computerprogramm mit Programmcode zur Durchführung des Verfahrens nach einem der vorhergehenden Verfahrensansprüche, wenn das Programm in einem Computer ausgeführt wird.

**Claims**

**1.** Method for cleaning and/or disinfecting an item (112) to be cleaned, wherein the item (112) to be cleaned is exposed to at least one cleaning fluid (116), heat equivalents to which the item (112) to be cleaned is exposed are recorded, the heat equivalents are added up, **characterized in that** the heat equivalents are additionally weighted with a compensation function during the summation of the heat equivalents, wherein the compensation function is a function of the temperature at which the heat equivalents were recorded, and the compensation function provides heat equivalents recorded in at least one exceptional temperature range (410) with a lower weighting than heat equivalents recorded in other temperature ranges and the exceptional temperature range (410) extends between a minimum temperature of a standard used for recording the heat equivalents and at least one predetermined regrowth temperature, the regrowth temperature being higher than the minimum temperature, and the compensation function is selected while taking into account at least one information item concerning at least one target germ, with which the item (112) to be cleaned could be contaminated.

**2.** Method according to the preceding claim, wherein the compensation function is adapted so that heat equivalents which have been recorded in the at least one exceptional temperature range (410) are not taken into account in the summation.

**3.** Method according to one of the preceding claims, wherein the compensation function assumes a value of less than one, in particular the value zero, for temperatures inside the exceptional temperature range (410), and the value one for temperatures outside the exceptional temperature range (410).

**4.** Method according to one of the preceding claims, wherein the regrowth temperature is selected so that delayed regrowth of apparently killed and/or only partially killed and/or at least temporarily inactivated test germs and/or at least one predetermined target germ takes place at least in a reduced fashion.

**5.** Method according to one of the preceding claims, wherein the regrowth temperature is selected in a temperature interval of between 67°C and 95°C, preferably in a temperature interval of between 70°C and 87°C, and particularly preferably in a temperature interval of between 75°C and 85°C.

**6.** Method according to one of the preceding claims, wherein the exceptional temperature range (410) is selected so that at least one temperature range not assigned to the exceptional temperature range (410) is arranged between a minimum temperature of a standard used for recording the heat equivalents and a starting temperature of the exceptional temperature range (410).

**7.** Method according to one of the preceding claims, wherein the heat equivalents are recorded according to the NSF3 standard or according to the EN ISO 15883 standard.

**8.** Method according to one of the preceding claims, wherein at least one target disinfection effect is specified, the target disinfection effect comprises a minimum sum of heat equivalents, and the method is carried out at least until the summation yields the minimum sum.

9. Method according to one of the preceding claims, wherein at least one gradient of the temperature is furthermore determined.

10. Method according to the preceding claim, wherein the compensation function is selected while taking the gradient into account.

11. Cleaning device (110) for cleaning and/or disinfecting an item (112) to be cleaned on the basis of the recording and accumulation of heat equivalents, contained in one or more cleaning chambers (114), having a nozzle system (122) for applying cleaning fluid (116) to the item (112) to be cleaned, and having one or more temperature sensors (124), wherein the cleaning device comprises at least one controller (126) for controlling the cleaning process having input and output means for a user and/or an external data processing device in order, in addition to the inputting of a compensation function for the weighting of the heat equivalents, to input one or more of the following values:

   - an exceptional temperature range (410)
   - a target germ with which the item (112) to be cleaned could be contaminated
   - a storage time to be expected after cleaning for the item (112) to be cleaned.

12. Computer program having program code for carrying out the method according to one of the preceding method claims, when the program is run on a computer.


**Revendications**

1. Procédé pour le nettoyage et/ou la désinfection d'un produit à nettoyer (112), où le produit à nettoyer (112) est soumis à l'effet d'au moins un fluide de nettoyage (116), les équivalents thermiques auxquels le produit à nettoyer (112) est soumis étant enregistrés et additionnés, **caractérisé en ce que** lors de l'addition des équivalents thermiques, ces derniers sont en outre pondérés par une fonction de compensation, la fonction de compensation étant une fonction de la température à laquelle les équivalents thermiques sont enregistrés et la fonction de compensation dotant les équivalents thermiques enregistrés dans au moins une plage de température exceptionnelle (410) d'une pondération moindre que les équivalents thermiques enregistrés dans les autres plages de température et la plage de température exceptionnelle (410) s'étendant entre une température minimale d'une norme utilisée pour l'enregistrement des équivalents thermiques et au moins une température de croissance prédéfinie, la température de croissance étant supérieure à la température minimale et la fonction de compensation étant choisie en tenant compte d'au moins une information relative à au moins un germe cible par lequel le produit à nettoyer (112) pourrait être contaminé.

2. Procédé selon la revendication précédente, la fonction de compensation étant aménagée de manière telle que les équivalents thermiques qui sont enregistrés dans ladite au moins une plage de température exceptionnelle (410) ne sont pas pris en considération dans l'addition.

3. Procédé selon l'une quelconque des deux revendications précédentes, la fonction de compensation ayant une valeur inférieure à un pour des températures dans la plage de température exceptionnelle (410), en particulier la valeur zéro, et la valeur un pour les températures en dehors de la plage de température exceptionnelle (410).

4. Procédé selon l'une quelconque des revendications précédentes, la température de croissance étant choisie de manière telle qu'une post- croissance retardée de germes test apparemment tués et/ou seulement partiellement tués et/ou au moins temporairement inactivés et/ou d'au moins un germe cible défini se réalise de manière au moins diminuée.

5. Procédé selon l'une quelconque des revendications précédentes, la température de croissance étant choisie dans un intervalle de température entre 67°C et 95°C, de préférence dans un intervalle de température entre 70°C et 87°C et de manière particulièrement préférée dans un intervalle de température entre 75°C et 85°C.

6. Procédé selon l'une quelconque des revendications précédentes, la plage de température exceptionnelle (410) étant choisie de manière telle qu'entre une température minimale d'une norme utilisée pour l'enregistrement des équivalents thermiques et une température de départ de la plage de température exceptionnelle (410) se trouve au moins une plage de température non associée à la plage de température exceptionnelle (410).

7. Procédé selon l'une quelconque des revendications précédentes, les équivalents thermiques étant enregistrés selon la norme NSF3 ou la norme EN ISO 15883.

8. Procédé selon l'une quelconque des revendications précédentes, au moins un effet de désinfection cible étant défini, l'effet de désinfection cible comprenant au moins une somme minimale d'équivalents thermiques, le procédé étant réalisé pendant un laps de temps au moins jusqu'à ce que la somme vaille la somme minimale.

9. Procédé selon l'une quelconque des revendications précédentes, au moins un gradient de la température étant en outre déterminé.

10. Procédé selon l'une quelconque des revendications précédentes, la fonction de compensation étant choisie en tenant compte du gradient.

11. Dispositif de nettoyage (110) destiné au nettoyage et/ou à la désinfection d'un produit à nettoyer (112), basé sur l'enregistrement et un cumul d'équivalents thermiques, enregistré dans une ou plusieurs chambres de nettoyage (114), présentant un système de pulvérisateurs (122) destiné à soumettre le produit à nettoyer (112) à un fluide de nettoyage (116) et un ou plusieurs capteurs de température (124), le dispositif de nettoyage comprenant au moins un dispositif de commande (126) pour le contrôle du processus de nettoyage avec des moyens d'introduction et d'émission pour un utilisateur et/ou un dispositif de traitement de données externe, afin d'introduire, en plus de l'introduction d'une fonction de compensation pour la pondération des équivalents thermiques, une ou plusieurs des grandeurs suivantes :

   - une plage de température exceptionnelle (410)
   - un germe cible par lequel le produit à nettoyer (112) pourrait être contaminé
   - une durée d'entreposage escomptée du produit à nettoyer (112) après le nettoyage.

12. Programme informatique composé d'un code de programmation destiné à réaliser le procédé selon l'une quelconque des revendications précédentes, lorsque le programme est exécuté dans un ordinateur.

FIG. 1

# FIG. 2 A

# FIG. 2B

```
                    ┌──────────────┐
              ┌────→│      ϑ        │〜── 210         ┌──────────────┐
              │     └──────────────┘                 │     228       │
              │            │                         └──────────────┘
              │            │                                │
              │      ┌─────┴─────┐             ┌────────────┤
              │      ▽           ▽             ▽            ▽
              │  ┌──────────┐          ┌──────────────┐
              │  │  W ( t )  │〜── 212   │   K (ϑ)       │〜── 226
              │  └──────────┘          └──────────────┘
              │      │                         │
              │      └──────┐      ┌───────────┘
┌──────────┐  │             ▽      ▽
│   Δ t     │〜── 220      ┌──────────────┐
└──────────┘  │           │   W * ( t )   │〜── 230
     △        │           └──────────────┘
     │        │                  │
     │        │                  ▽
     │        │           ┌──────────────┐
     │        │           │  Aₒ * ( t )   │〜── 232
     │        │           └──────────────┘
     │        │                  │
     │        │                  ▽
 218 │        │           ╱──────────────╲
     │        └──────────╱  Aₒ*( t ) ≥ Aₒᶻ ?╲〜── 234
     └───────────────────╲                ╱
                          ╲──────────────╱
                                 │〜── 222
                                 ▽
                          ┌──────────────┐
                          │     224       │
                          └──────────────┘
```

# FIG. 3

FIG. 4 A

FIG. 4 B

FIG. 5 A

FIG. 5 B

**IN DER BESCHREIBUNG AUFGEFÜHRTE DOKUMENTE**

**In der Beschreibung aufgeführte Patentdokumente**

- WO 2006097294 A1
- DE 102007021245 A1